(19) Europäisches Patentamt
European Patent Office
Office européen des brevets

(11) Veröffentlichungsnummer: **0 118 808**
**B1**

(12) # EUROPÄISCHE PATENTSCHRIFT

(45) Veröffentlichungstag der Patentschrift: **22.11.90**

(51) Int. Cl.$^5$: **C 12 P 21/02**

(21) Anmeldenummer: **84101814.6**

(22) Anmeldetag: **21.02.84**

(54) **Reinigung von Interferon.**

(30) Priorität: **03.03.83 CH 1151/83**
**13.12.83 CH 6642/83**

(43) Veröffentlichungstag der Anmeldung:
**19.09.84 Patentblatt 84/38**

(45) Bekanntmachung des Hinweises auf die Patenterteilung:
**22.11.90 Patentblatt 90/47**

(84) Benannte Vertragsstaaten:
**AT BE CH DE FR GB IT LI LU NL SE**

(56) Entgegenhaltungen:
**EP-A-0 011 435**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE AG**
**Postfach 3255**
**CH-4002 Basel (CH)**

(72) Erfinder: **Hochuli, Erich, Dr.**
**Kirchackerstrasse 25**
**CH-4411 Arisdorf (CH)**

(74) Vertreter: **Lederer, Franz, Dr. et al**
**Van der Werth, Lederer & Riederer**
**Patentanwälte Lucile-Grahn-Strasse 22**
**D-8000 München 80 (DE)**

(56) Entgegenhaltungen:
**JOURNAL OF CHROMATOGRAPHY, Band 198, 1980, Seiten 247-255, Elsevier Publishing Company, Amsterdam, NL; P. HUBERT et al.: "Chrom. 13,023. Metal chelate affinity chromatography. I. Influence of various parameters on the retention of nucleotides and related compounds"**

**CHEMICAL ABSTRACTS, Band 95, Nr. 15, 12. Oktober 1981, Seite 506, Zusammenfassung Nr. 130856f, Columbus, Ohio, US**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 118 808 B1

(56) Entgegenhaltungen:

CHEMICAL ABSTRACTS, Band 95, Nr. 15, 12. October 1981, Seite 506, Zusammenfassung Nr. 130857g, Ohio, US

CHEMICAL ABSTRACTS, Band 95, Nr. 15, 12. Oktober 1981, Seite 506, Zusammenfassung Nr. 130858h, Columbus, Ohio, US

**Beschreibung**

Die vorliegende Erfindung betrifft ein Verfahren zur Reinigung rekombinanter Interferone mittels Metallchelat-Chromatographie sowie die Verwendung bestimmter Metallchelatharze zur Reinigung rekombinanter Interferone.

Unter Interferonen versteht man eine Gruppe körpereigener Proteine mit antiviraler und immunregulatorischer Wirkung. Der antivirale Effekt wird dabei nicht durch eine direkte Beeinflussung der Viren selbst erzielt sondern durch eine Wirkung auf deren Zielzellen im Sinne eines Schutzes gegen die Virusinfektion. Die Interferone können objektivierbare Effekte auf Krebsgeschwülste ausüben, die sie für den Einsatz in der Krebstherapie geeignet machen und beeinflussen das körpereigene Immunsystem, indem sie z.B. Makrophagen und NK-Zellen aktivieren und die Expression verschiedener immunologisch bedeutsamer Bestandteile der Zellmembran verstärken.

Humane Interferone (α, β und γ) können heute dank rekombinanter DNA-Technologie auf mikrobiellem Wege in Mengen hergestellt werden, die trotz grösster Anstrengungen durch Isolierung aus natürlichem Material (Leukocyten, Fibroblasten, Lymphocyten) und Reinigung nicht zur Verfügung gestellt werden können.

Erst diese neue Technologie hat daher einen Weg für die intensive klinische Erprobung und allfällige breite therapeutische Anwendung der Interferone geöffnet und lässt eine hinreichende Versorgung der für eine Behandlung infrage kommenden Personen mit den Wirksubstanzen möglich erscheinen.

Einzelheiten der Klonierung von Interferon-cDNA und deren direkter Expression, insbes. in E. coli, sind inzwischen vielfach publiziert worden. So ist die Herstellung rekombinanter Interferone beispielsweise bekannt aus J. Interferon Res. *1* (1981), 381—390 (Wetzel et al.), Nature *284* (1980), 316—320 (Nagata et al.), Nucleic Acids Res. *8* (1980), 4057—4074 (Goeddel et al.), Nucleic Acids Res. *10* (1982), 2487—2501 (Devos et al.), Nature *295* (1982), 503—508 (Gray et al.), sowie aus den deutschen Offenlegungsschriften Nrn. 31 25 706, 31 38 096 und 31 44 469.

Da die rekombinanten Interferone mikrobiellen Ursprungs sind (z.Z. stammen sie vorzugsweise aus E. coli) sind sie nach ihrer Isolierung aus dem Mikroorganismus bzw. dem Kulturmedium zunächst noch durch eine Reihe mikrobieller Begleitsubstanzen kontaminiert, deren Anwesenheit für eine therapeutische Anwendung der so gewonnenen Interferone prohibitiv ist. Die Reinigung des rekombinanten Materials spielt daher eine besonders wichtige Rolle. Zur Reinigung rekombinanter Interferone wurde bisher eine Vielzahl unterschiedlicher Methoden, insbesondere chromatographische, herangezogen und miteinander kombiniert (siehe z.B. deutsche Offenlegungsschrift Nr. 31 25 706). Dabei hat sich vor allem die Chromatographie an Immunadsorbentien, also an anti-Interferon-Antikörpern, als wertvolles Hilfsmittel herausgestellt. So wurde die Reinigung rekombinanten Human-Leukocyten-Interferons (HuIFN-α) mittels monoklonaler Antikörper beispielsweise von Staehelin et al. (J. Biol. Chem. *256* (1981), 9750—9754) und von Secher et al. (Nature *285* (1980), 446—450) beschrieben. Aufgrund der hohen Spezifität dieser Immunadsorbentien durfte man davon ausgehen, dass das so gereinigte Material praktisch frei von kontaminierenden Substanzen ist und einen hohen Reinheitsgrad aufweist.

Bei der Reinigung grösserer Mengen rekombinanten Leukocyten-Interferons mittels monoklonaler Antikörper stellte sich jedoch heraus, dass das gereinigte Material sowohl Interferon-Bruchstücke (Interferon, dem ein Teil der aminoendständigen Aminosäuresequenz fehlt) wie Interferon-Oligomere, beispielsweise Dimere, enthält. Diese unerwünschten Nebenprodukte besitzen bei vergleichbarer Affinität zum Antikörper nur noch einen Teil der biologischen Aktivität des reinen Interferons.

Nun ist in der europäischen Patentschrift Nr. 11435 eine Methode zur Reinigung von Human-Fibroblasten-Interferon (HuIFN-β) mittels eines aus Nature *258* (1975), 598—599 (Porath et al.) bekannten Harzes der Formel

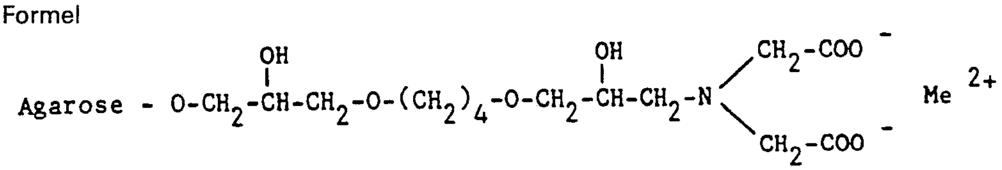

worin Me Kobalt, Nickel, Zink oder Kupfer bedeutet, beschrieben worden. Auch Edy et al. (J. Biol. Chem. *252* (1977), 5934—5935) haben die erfolgreiche Reinigung von HuIFN-β an dem obenbeschriebenen Zinkchelatharz beschrieben. Chadha et al, wiesen dann nach (J. gen. Virol. *43* (1979), 701—706), dass HuIFN-α nicht in analoger Weise an den bei HuIFN-β erfolgreich eingesetzten Zink- oder Kupferchelatharzen gereinigt werden kann und bestätigten damit die bereits von Edy et al. (loc. cit.) gemachte Beobachtung.

Erfindungsgemäss wurde nun gefunden, dass die Reinigung rekombinanter Interferone, gleichgültig ob IFN-α, IFN-β oder IFN-γ, gelingt, wenn man statt des von Porath et al. beschriebenen Metallchelatharzes zu einem prinzipiell ähnlich gebauten Harz der Formel

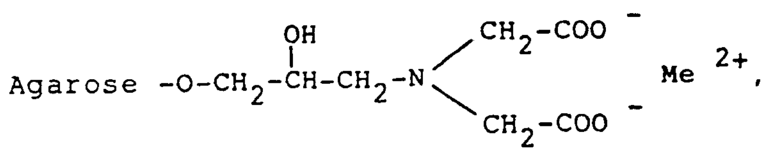

wobei Me Kupfer oder Nickel bedeutet, übergeht, das sich also durch einen kürzeren Spacer zwischen Agarose-Matrix und Iminodiessigsäurerest auszeichnet. Dieses Harz und seine Herstellung ist bereits bekannt, z.B. aus J. Chromatography *198* (1980), 247—255 (Hubert und Porath). Die Autoren beschrieben aber seine Verwendbarkeit ausschliesslich für die Fraktionierung von Oligo- und Polynucleotiden, einer Verbindungsklasse, die sich von Interferonen so stark unterscheidet, dass es für den Fachmann in keiner Weise naheliegend war, es für die Lösung des vorliegenden Problems der Reinigung von rekombinanten Interferonen, insbesondere deren Abtrennung von sie begleitenden sequenzhomologen Bruchstücken und Oligomeren, heranzuziehen.

Die vorliegende Erfindung betrifft daher ein Verfahren zur Reinigung rekombinanter Interferone, das dadurch gekennzeichnet ist, dass man eine vorgereinigte Interferon-Lösung mit einem Metallchelatharz folgender Struktur

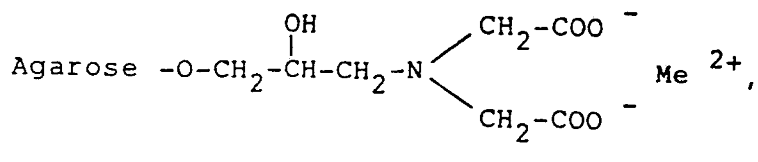

wobei Me Kupfer oder Nickel darstellt, in Kontakt bringt und das Interferon in reinerer Form durch Behandlung des beladenen Harzes mit einer Waschflüssigkeit eluiert sowie die Verwendung dieses Metallchelatharzes zur Reinigung rekombinanter Interferone.

Das erfindungsgemäss verwendbare Metallchelatharz kann in bekannter Weise — wie z.B. von Hubert und Porath in J. Chromatog. *198* (1980), 247 beschrieben — aus Agarose durch Behandlung mit Epichlorhydrin, Umsetzung des erhaltenen Epoxids mit Iminodiessigsäure-dinatriumsalz und Ueberführung in das Kupfer- oder Nickelsalz durch Waschen mit einer Kupfer (II)- oder Nickelsalzlösung, beispielsweise mit Kupfersulfat oder Nickelchlorid, hergestellt werden. Statt Epichlorhydrin kann auch Epibromhydrin eingesetzt werden. Als Agarose verwendet man zweckmässigerweise ein standardisiertes Produkt, vorzugsweise Sepharose® der Firma Pharmacia, Uppsala, Schweden. Besonders bevorzugt ist Sepharose® 4B.

Im folgenden ist die Herstellung eines geeigneten Kupferchelatharzes im Detail wiedergegeben:

1 l Agarosegel (Sepharose® 4B, Pharmacia) wurde auf einer Glasfritte zweimal mit je 1 l Wasser gewaschen, in ein 4 1-Reaktionsgefäss überführt und mit Wasser auf ein Volumen von 2000 ml gebracht. Nach Zusatz von 150 ml 4N NaOH und 40 ml Epibromhydrin wurde das Gemisch 5 Stunden bei 30°C gerührt. Das aktivierte Harz wurde auf einer Glasfritte mit Wasser neutralgewaschen, mit 650 ml wässriger 2N $Na_2CO_3$-Lösung versetzt und mit Wasser auf ein Volumen von 2000 ml gebracht. Dann setzte man 100 g Iminodiessigsäure-dinatriumsalz zu und rührte das Gemisch 20 Stunden bei 65°C. Anschliessend wurde das Harz in einer Säule nacheinander mit je 2000 ml Wasser, wässrigem $CuSO_4 \cdot 5H_2O$ (0,5 Gew.-%), Wasser, 0,2 M Essigsäure (0,2 M NaCl, 0,1 Gew./Vol.% Tween 20), Wasser gewaschen. Die Kupferionenkonzentration betrug etwa 9 Mikromol/ml Harz.

Die Metallchelat-Säule wird vor der Beladung mit Interferon zweckmässigerweise mit einem wässrigen Puffer (pH ca. 5—8), der selber keine Chelate mit Kupfer oder Nickel bildet, vorzugsweise einem Phosphatpuffer pH 7, äquilibriert. Der Aequilibrierungspuffer (wie auch die Elutionspuffer) kann einen Stabilisator bzw. Emulgator, beispielsweise vom Polyoxyethylen-Fettalkoholether-Typ, Polyoxyethylen-Sorbitan-Fettsäureester-Typ oder Triton, enthalten. Der Zusatz eines solchen Stabilisators ermöglicht problemloses Arbeiten auch bei höheren Interferon-Konzentrationen.

In analoger Weise kann bei Verwendung eines Nickelsalzes, z.B. Nickelchlorid, das Nickelchelatharz hergestellt werden:

Die Elution erfolgt mit wässrigen, mit Kupfer oder Nickel nicht chelatisierenden Pufferlösungen in an sich bekannter Weise, vorzugsweise mit Phosphat- oder Acetatpuffern, wobei die Retention der Interferon-Bruchstücke, der monomeren Interferone und der Oligomeren eine Funktion des pH-Werts und der Ionenstärke ist. Mit fallendem pH-Wert und steigender Ionenstärke werden zunächst die Interferon-Bruchstücke, dann das monomere Interferon und schliesslich, beispielsweise mit verdünnter Essigsäure, die Oligomeren eluiert. Innerhalb bestimmter, dem Fachmann geläufiger Grenzen gilt, dass je höher die Ionenstärke des Etulionspuffers gewählt wird, desto höher kann auch sein pH-Wert sein. Die Ionenstärke des Puffers kann durch Zusatz neutraler Salze wie NaCl erhöht werden. Bei geeigneter Einstellung des pH-Werts der die Bruchstücke und Oligomere enthaltenden Interferonlösung, beispielsweise auf pH ca. 5, werden nur das Monomere und die Oligomeren adsorbiert, während die Bruchstücke durchfliessen.

Die Elution kann bei konstantem pH-Wert oder mit linear oder diskontinuierlich fallendem pH-Gradienten durchgeführt werden. Die optimalen Elutionsbedinungen hängen von der Menge und Art der vorhandenen Verunreinigungen, der Menge des zu reinigenden Materials, den Säulendimensionen usw. ab und sind zweckmässigerweise von Fall zu Fall zu bestimmen.

Enthalten die Elutionpuffer Stabilisatoren, so können diese durch Chromatographie der Eluate an

geeigneten Trägern, beispielsweise an Cellulose, entfernt werden. Erfindungsgemäss gereinigtes, Interferon kann schliesslich aus Polyethylenglykol/Wasser kristallisiert werden.

Die Reinigung von Leukocyten-Interferon erfolgt vorzugsweise an der Kupferchelatsäule, die von Fibroblasten-Interferon vorzugsweise an der Nickelchelatsäure.

Die folgenden Beispiele illustrieren das erfindungsgemässe Verfahren.

Das als Ausgangsmaterial verwendete rekombinante Human-Leukozyten-Interferon A (rIFN-αA) wurde durch Reinigung an monoklonalen Antikörpern nach der von Staehelin et al. beschriebenen Methode erhalten (J. Biol. Chem. *256* (1981), 9750—9754).

Das als Ausgangsmaterial verwendete rekombinante Human-Fibroblasten-Interferon (rIFN-β), hergestellt nach der Methode von Goeddel et al. (Nucleic Acids Res. *8*, 4057—4074 [1980]), wurde durch Chromatographie an immobilisierten Triazinfarbstoffen (z.B. Blue-Sepharose CL-6B® der Firma Pharmacia oder Blue Trisacryl M® der Firma LKB) vorgereinigt nach der Methode von Friesen et al. (Arch. Biochem, Biophys. *206*, 432—450 [1981]).

Die Bestimmung des Proteingehaltes erfolgte nach der Methode von Lowry et al. (J. Biol. Chem. *193* (1951), 265—275) unter Verwendung von Serumalbumin als Standard.

Die quantitative Bestimmung der Verunreinigungen (Fragmente und Oligomere) erfolgte mittels SDS—PAGE wie von Laemmli et al. beschrieben (Nature *277* (1970), 680—685), mit der Modifikation, dass die Elektrophorese unter nicht-reduzierenden Bedingungen (d.h. ohne Zusatz von 2-Mercapto-ethanol zur Probe) durchgeführt wurde.

### Beispiel 1

Eine Kupferchelat-Säule (169 ml, 5 × 8,5 cm) wurde mit 500 ml Phosphatpuffer (0,05 M, pH 7,0, 0,1 Gew./Vol.-% Tween 20) äquilibriert. Die Säule wurde bei 4°C beladen mit 1480 ml eines gemäss Staehelin et al. (loc. cit.) erhaltenen Eluats aus einer monoklonalen rIFN-αA-Antikörper-Säule, das 0,28 mg/ml Protein enthielt (zu 23,5 Gew.-% kontaminiert mit einem 15 kd-Interferon-Bruchstück) und mit 4N NaOH auf pH 7 eingestellt worden war. Die Säule wurde nacheinander gewaschen mit 300 ml Aequilibrierungspuffer, 300 ml Aequilibrierungspuffer, zusätzlich enthalten 0,2M NaCl, sowie mit 300 ml 0,05M Acetatpuffer pH 5,6 (enthaltend 0,2M NaCl und 0,1% Tween 20). Zunächst wurde das Interferon-Bruchstück mit einem 0,05M Acetatpuffer (enthaltend 0,2M NaCl und 0,1% Tween 20) bei einem von 5,6 auf 4,0 fallenden pH-Gradienten ausgewaschen. Dann wurden 256 mg monomeres Interferon (Reinheit > 95%) mit dem gleichen Acetatpuffer, pH 4,0, eluiert.

Die 0,1 Gew./Vol.-% Tween enthaltende Lösung des monomeren Interferons wurde zwecks Entfernung des Stabilisators an einer CM 52-Cellulose-Säule mit einem 0,1M Ammoniumacetat-Puffer (pH 5) chromatographiert.

### Beispiel 2

Die gleiche äquilibrierte Kupferchelat-Säule wie in Beispiel 1 wurde bei 4°C beladen mit 2168 ml eines gemäss Staehelin et al. (loc. cit.) erhaltenen Eluats aus einer monoklonalen rIFN-αA-Antiköroper-Säule, das 0,33 mg/ml Protein enthielt (zu 27 Gew.-% kontaminiert mit 15 Kd Interferon-Bruchstücken) und mit 4N NaOH auf pH 4,5 eingestellt worden war. Die Säule wurde dann mit einem 0,05M Acetatpuffer (pH 5,6; enthaltend 0,2M NaCl und 0,1% Tween) gewaschen. Unter diesen Bedingungen wurde nun das komplette 18,5 Kd rIFN-αA, nicht aber das 15 Kd Bruchstück, adsorbiert und anschliessend mit einem 0,05M Acetatpuffer (pH 4; 0,2M NaCl und 0,1% Tween 20) eluiert. Es wurden 402 mg Interferon mit einer Reinheit 95% erhalten.

### Beispiel 3

Die gleiche äquilibrierte Kupferchelat-Säule wie in Beispiel 1 wurde bei 4°C beladen mit 1260 ml eines gemäss Staehelin et al. (loc. cit.) erhaltenen Eluats aus einer monoklonalen rIFN-αA-Antikörper-Säule, das 0,5 mg/ml Protein enthielt (zu 34 Gew.-% kontaminiert mit Interferon-Oligomeren: einem 37 Kd-Dimeren, einem 55,5 Kd-Trimeren und einem 74 Kd-Tetrameren) und mit 4N NaOH auf pH 7 eingestellt worden war. Die Säule wurde zunächst mit 300 ml eines 0,05M Phosphatpuffers (pH 7; 0,1% Tween) gewaschen und anschliessend mit einem 0,05M Acetatpuffer (pH 4,7; enthaltend 0,2M NaCl und 0,1% Tween 20) eluiert, wobei monomeres Interferon mit einer Reinheit von 95% erhalten wurde. 150 mg dimeres Interferon wurde mit 0,05M Acetatpuffer vom pH 4,0 (enthaltend 0,2M NaCl und 0,1% Tween 20) eluiert, die höheren Oligomeren mit 0,2M Essigsäure (enthaltend 0,2M NaCl und 0,1% Tween 20).

### Beispiel 4

Eine Kupferchelat-Säule (295 ml, 5 × 15 cm) wurde mit 600 ml Phosphatpuffer (0,05M, pH 7, 0,1% Tween 20) äquilibriert. Die Säule wurde bei 4°C mit 3200 ml eines gemäss Staehelin et al. (loc. cit.) erhaltenen Eluats aus einer monoklonalen rIFN-αA-Antikörper-Säule, das 0,33 mg/ml Protein enthielt (kontaminiert zu 24 Gew.-% mit einem 15 Kd-Interferon-Bruchstück und zu 11 Gew.-% mit Interferon-Oligomeren), beladen. Durch Waschen mit einem 0,005M Acetatpuffer (pH 4; 0,025M NaCl, 0,1% Tween 20) wurde das 15 Kd-Bruchstück entfernt. Monomeres Interferon wurde mit 0,025M Acetatpuffer (pH 3,4; 0,025M NaCl, 0,1% Tween 20) eluiert, wobei insgesamt 470 mg mit einer Reinheit > 95% erhalten wurden. Schliesslich wurden die Oligomeren mit 0,2M Essigsäure (0,2M NaCl, 0,1% Tween 20) eluiert.

Beispiel 5

Eine Kupferchelat-Säule (1000 ml, 10 × 13 cm) wurde mit 2500 ml Phosphatpuffer (0,05M, pH 5, 0,2M NaCl, 0,1% Tween 20) äquilibriert und bei 4°C mit 10 000 ml eines gemäss Staehelin et al. (loc. cit.) erhaltenen Eluats aus einer monoklonalen rIFN-αA-Antikörper-Säule, das 0,21 mg/ml Protein enthielt (kontaminiert zu 3 Gew.-% mit einem 15 Kd-Interferon-Bruckstück und zu 7 Gew.-% mit Interferon-Oligomeren) und mit 6N NaOH auf pH 7 eingestellt worden war, beladen. Das Fragment wurde durch Waschen mit dem Aequilibrierungspuffer entfernt. 1700 mg monomeres Interferon (Reinheit > 95%) wurden mit, 0,05M Acetatpuffer (pH 4,5, 0,2M NaCl, 0,1% Tween 20) eluiert. Die Oligomeren wurden durch Elution mit 0,2M Essigsäure (0,2M NaCl, 0,1% Tween 20) erhalten. Die Lösung des monomeren Interferons wurde zur Konzentration und Entfernung der 0,1 Gew./Vol.-% Tween 20 an einer CM 52-Cellulose-Säule mit 0,1M Ammoniumacetatpuffer (pH 5) chromatographiert.

Zu 195 ml des Eluats der Cellulose-Säule, enthaltend 4 mg/ml Protein, wurden bei 4°C unter leichtem Rühren langsam 48,7 ml einer wässrigen Lösung von Polyethylenglykol 4000 (0,3 g/ml) gegeben. Nach etwa 5 Stunden bildeten sich die ersten Kristalle, die nach 3 Tagen in der Kälte durch Zentrifugieren vom Ueberstand abgetrennt, mit kaltem wässrigem Polyethylenglykol 4000 (0,3 g/ml) gewaschen und unter vermindertem Druck getrocknet wurden. Ausbeute: 520 mg. Mittels Gelelektrophorese und Bioassay einer durch Lösen einiger Kristalle in 0,1M Ammoniumacetatpuffer (pH 5) erhaltenen Probe konnte nachgewiesen werden, dass es sich bei den erhaltenen Kristallen um reines intaktes rIFN-αA handelte.

Beispiel 6

Die Kupferionen einer Kupferchelatsäule (12 ml, 1.6 × 6 cm) wurden mit 20 ml einer 0,1 molaren wässrigen Lösung von Ethylendiamin-tetraessigsäure-dinatriumsalz und dann mit 100 ml Wasser ausgewaschen. Anschliessend wurde die Säule nacheinander mit je 36 ml, 0,05 M $NiCl_2 \cdot 6H_2O$ in Wasser, Wasser, 0,05 M Essigsäure (enthaltend 0,5 M NaCl) und Wasser gewaschen.

Die so erhaltene Nichelchelatsäule wurde mit 36 ml Phosphatpuffer (0,05 M, pH 7,2, 10 Gew./Vol.-% Ethylenglykol, 0,2 M NaCl) äquilibriert. 38 ml eines gemäss Goeddel et al./Friesen et al. (loc. cit.) erhaltenen Eluats von einer Blue-Sepharose®-Säule, das 0,12 mg/ml Protein enthielt, wurde mit 152 ml Phosphatpuffer (0,05M, pH 7.2) verdünnt und bei 4°C auf die Nickelchelatsäule aufgegeben. Die Säule wurde mit 72 Acetatpuffer (0,05 M, pH 7, 10 Gew./Vol.-% Propylenglykol, 0,2 M NaCl) gewaschen und dann mit Acetatpuffer (0,05 M, pH 3,5, 10 Gew./Vol.-% Propylenglykol, 0,3 M NaCl) eluiert. Es wurden 3,75 mg reines (Reinheit > 95%) Fibroblasten-Interferon erhalten. Anschliessend wurde die Säule mit 0,05 M Essigsäure (enthalten 0,5 M NaCl) gewaschen und war wieder einsatzbereit.

**Patentansprüche**

1. Verfahren zur Reinigung rekombinanter Interferone, dadurch gekennzeichnet, dass man eine vorgereinigte Interferon-Lösung mit einem Metallchelatharz folgender Struktur

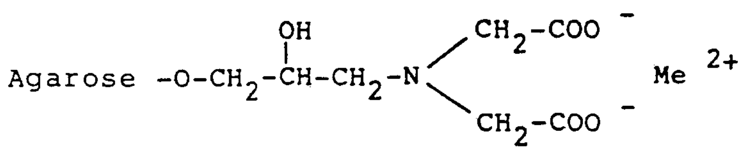

wobei Me Kupfer oder Nickel darstellt, in Kontakt bringt und das Interferon in reinerer Form durch Behandlung des beladenen Harzes mit einer Waschflüssigkeit eluiert.

2. Verfahren gemäss Anspruch 1, dadurch gekennzeichnet, dass ein Kupferchelatharz verwendet wird.

3. Verfahren gemäss Anspruch 1, dadurch gekennzeichnet, dass ein Nickelchelatharz verwendet wird.

4. Verfahren gemäss Anspruch 2 oder 3, dadurch gekennzeichnet, dass es sich bei der Agrose um Sepharose® 4B handelt.

5. Verfahren gemäss Anspruch 2, dadurch gekennzeichnet, dass rekombinantes Leukocyten-Interferon gereinigt wird.

6. Verfahren gemäss Anspruch 3, dadurch gekennzeichnet, dass rekombinantes Fibroblasten-Interferon gereinigt wird.

7. Verfahren gemäss Anspruch 2, dadurch gekennzeichnet, dass das reinere Interferon durch Gradientelution erhalten wird.

8. Verfahren gemäss den Ansprüchen 5 und 7, dadurch gekennzeichnet, dass die Elution mit einem von 5,6 von 4,0 abfallenden pH-Gradienten erfolgt.

9. Verfahren gemäss Anspruch 3, dadurch gekennzeichnet, dass die Elution mit einem Acetatpuffer pH 3.5 erfolgt.

**Revendications**

1. Procédé pour purifier des interférons recombinants, caractérisé en ce que l'on met une solution

d'interféron ayant subi une purification préalable en contact avec un chélate métallique résineux possédant la structure suivante:

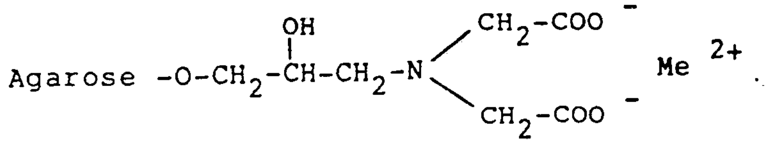

dans laquelle Me représente le cuivre ou le nickel, et on élue l'interféron plus pur par traitement de la résine chargée à l'aide d'un liquide de lavage.

2. Procédé selon la revendication 1, caractérisé en ce que l'on utilise un chélate résineux de cuivre.

3. Procédé selon la revendication 1, caractérisé en ce que l'on utilise un chélate résineux de nickel.

4. Procédé selon la revendication 2 ou 3, caractérisé en ce que l'agarose utilisée est le produit du commerce Sepharose 4B.

5. Procédé selon la revendication 2, caractérisé en ce que l'on purifie de l'interféron recombinant de leucocytes.

6. Procédé selon la revendication 3, caractérisé en ce que l'on purifie de l'interféron recombinant de firboblastes.

7. Procédé selon la revendication 2, caractérisé en ce que l'on obtient l'interféron plus pur par une élution en gradient.

8. Procédé selon les revendications 5 et 7, caractérisé en ce que l'on effectue l'élution à un gradient de pH diminuant de 5,6 à 4,0.

9. Procédé selon la revendication 3, caractérisé en ce que l'on élue à l'aide d'un tampon à l'acétate, pH 3,5.

**Claims**

1. A process for the purification of recombinant interferons, characterized by bringing a pre-purified interferon solution into contact with a metal chelate resin of the following structure

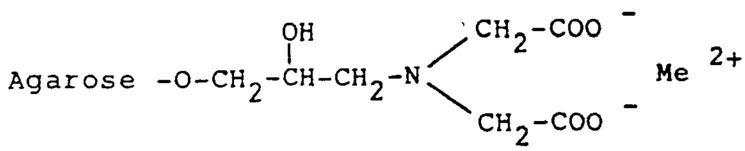

in which Me signifies copper or nickel, and eluting the interferon in purer form by treating the loaded resin with a washing liquid.

2. A process according to claim 1, characterized in that a copper chelate resin is used.

3. A process according to claim 1, characterized in that a nickel chelate resin is used.

4. A process according to claim 2 or 3, characterized in that the agarose is Sepharose® 4B.

5. A process according to claim 2, characterized in that recombinant leucocyte interferon is purified.

6. A process according to claim 3, characterized in that recombinant fibroblast interferon is purified.

7. A process according to claim 2, characterized in that the purer interferon is obtained by gradient elution.

8. A process according to claims 5 and 7, characterized in that the elution is carried out with a pH gradient falling from 5.6 to 4.0.

9. A process according to claim 3, characterized in that the elution is carried out with an acetate buffer of pH 3.5.